# EUROPEAN PATENT APPLICATION

(11) **EP 4 513 171 A1**
(43) Date of publication of application: **26.02.2025**
(21) Application number: 23791884.2
(22) Date of filing: 19.04.2023
(51) Int. Cl.: G01N 21/3577, G01N 21/359

(54) **FLUID CONCENTRATION MEASURING DEVICE**

(30) Priority: 19.04.2022 JP 2022068999
(71) Applicant: Nipro Corporation, Osaka 566-8510 (JP); Harada Electronics Industry Co., Ltd., Sapporo-shi, Hokkaido 063-0052 (JP)
(72) Inventor: SANO Yoshihiko, Settsu-shi, Osaka 566-8510 (JP); HARADA Masahide, Sapporo-shi, Hokkaido 063-0052 (JP)
(74) Representative: Mewburn Ellis LLP
(86) International application number: PCT/JP2023/015574
(87) International publication number: WO 2023/204236

(57) **Abstract**

The present invention makes it possible to easily and highly precisely measure the concentration of a fluid flowing inside a conduit. A fluid concentration measuring device of the invention includes a case having a groove with a constant width that receives a conduit in such a manner that the conduit is compression-deformed in the diametrical direction thereof with a passage being left thereinside; a light diffusion block for light projection that has a light projection surface positioned on the same plane as one of two side walls of the groove on one axis line which is orthogonal to the two side walls of the groove; a light diffusion block for light reception that has a light reception surface which is positioned on the same plane as the other of the two side walls on the axis line and faces the light projection surface; a light emitting element that is configured to supply transmission lights having two types of wavelengths to a given region which is part of the light projection surface of the light diffusion block for light projection; a light receiving element that is configured to be supplied with the transmission lights having the two types of wavelengths from a given region which is part of the light reception surface of the light diffusion block for light reception; a transmission light intensity measuring unit that is configured to measure, via a signal from the light receiving element, the intensities of the transmission lights with the two types of wavelengths; and a fluid concentration output unit that is configured to obtain and output the concentration of the fluid based on the intensities of the transmission lights with the two types of wavelengths, and on separately obtained input intensities thereof.

## Description

### Technical field

The present invention relates to a fluid concentration measuring device for measuring the concentration of a fluid inside a conduit that is light transmissive and deformable.

### Background Art

As a conventional fluid concentration measuring device, there is known, for example, a measuring device disclosed in Patent Literature 1. The measuring device disclosed in this literature is one used to measure the concentration of a process liquid as a fluid for washing semiconductor wafers, in which a plurality of measuring bodies are provided at midway portions of a process liquid supply pipe, light transmission parts with different optical path lengths of the light passing through the process liquid are respectively provided in the measuring bodies, a light from a light source is supplied to a light transmission part of an optical path length corresponding to the property of the process liquid, the light that has passed through the process liquid at such light transmission part is received by a light detector to have the intensity of such light examined, whereafter the concentration of the process liquid is obtained from such light intensity based on the Lambert-Beer law.

However, with the conventional device described above, if applied to measuring the concentration of a fluid such as blood or drug solution inside a light transmissive conduit such as a resin tube, light needs to be passed through an optical path crossing the light transmissive conduit. Here, it is difficult to actually measure the inner diameter of the conduit and the wall thickness of the conduit that serve as the optical path length; particularly, in the case of a resin tube with a deformable conduit, the inner diameter may change upon deformation of the tube, which makes it extremely difficult to measure the concentration of, for example, blood or a drug solution under such condition, thus making the measurements thereof practically impossible under the conventional setting.

Therefore, as shown in Patent Literature 2, the inventors of the present application have proposed a fluid concentration measuring device that is configured in the following manner. Specifically, there is provided a groove extended in a given direction along the upper surface of a case. Further, there are provided two pairs of light emitting unit and light receiving unit that differ from each other in optical path distance, of which the light emitting units and the light receiving units are respectively fixed to side wall portions of the groove in such a way that the corresponding light emitting unit and light receiving unit face each other, and a resin tube is sandwiched between the pairs of light emitting unit and light receiving unit while being compression-deformed in the diametrical direction. With regard to each optical path distance, a light is emitted from the light emitting unit and received by the light receiving unit, whereby the concertation of a fluid inside the resin tube can be obtained from the intensity of such light received based on the Lambert-Beer law.

### Citation List

### Patent Literature

Patent Literature 1: JP-A-H10-325797
Patent Literature 2: WO2014/170985

### Summary of Invention

### Technical Problem

With this conventional fluid concentration measuring device, it is critical to reliably bring the resin tube into close contact with the light emitting units and the light receiving units for the sake of precise concentration measurement. In this regard, the device is configured in such a manner where narrow segments are formed by the light emitting units and the light receiving units, and the resin tube is to be compression-deformed by such narrow segments. However, it is difficult to properly compression-deform the resin tube and place it into the narrow segments of the groove; and due to a narrow light transmission area, measurement errors may occur when there are scars on the surface of the resin tube and the sites of the scars vary. Thus, it was made clear that the device still had room for improvement. Further, even when removing the resin tube from the device after the measurement is over, the resin tube may get stuck in the narrow segments of the groove, and problems may thus occur in the resin tube and in the periphery of the groove if forcedly pulling the resin tube.

### Solution to Problem

The purpose of this invention is to advantageously solve the abovementioned problems. The fluid concentration measuring device of the present invention is a device for measuring the concentration of a fluid flowing in a light transmissive conduit that has a uniform wall thickness and outer diameter in a longitudinal direction thereof when in a free state and is elastically compression-deformable in a diametrical direction thereof. This fluid concentration measuring device includes:
a case having a groove that is extended in a given direction and has a portion with a constant groove width in at least a part of the groove in a longitudinal direction thereof, the portion with a constant groove width receiving the conduit in such a manner that the conduit is compression-deformed in the diametrical direction with a passage being left thereinside;
a light diffusion block for light projection that has a light projection surface positioned on the same plane as one of two side walls of the groove on one axis line which is orthogonal to the two side walls of the groove at the portion with the constant groove width;
a light diffusion block for light reception that has a light reception surface which is positioned on the same plane as the other of the two side walls on the axis line and faces the light projection surface with a given distance equal to the groove width being provided between the light reception surface and the light projection surface;
a light emitting element that is configured to supply transmission lights having two types of wavelengths to a given region which is a part of the light projection surface of the light diffusion block for light projection and is centered about the axis line, the two types of wavelengths being a first wavelength that yields an extremely small or absolutely zero absorbance to water but a high absorbance to the fluid, and a second wavelength that yields a high absorbance to water;
a light receiving element that is configured to be supplied with the transmission lights having the two types of wavelengths from a given region which is a part of the light reception surface of the light diffusion block for light reception and is centered about the axis line;
a transmission light intensity measuring unit that is configured to measure, via a signal from the light receiving element, the intensities of the transmission lights with the two types of wavelengths that are supplied from the light emitting element, projected from the light projection surface, received from the light reception surface, and then supplied to the light receiving element; and
a fluid concentration output unit that is configured to obtain and output the concentration of the fluid based on the intensities of the transmission lights with the two types of wavelengths that have passed through the conduit between the light projection surface and the light reception surface, and on separately obtained input intensities of the transmission lights of the two types of wavelengths to the conduit.

### Advantageous Effects of Invention

In the case of the fluid concentration measuring device of the present invention, when measuring the concentration of a fluid flowing in the light transmissive conduit that has a uniform wall thickness and outer diameter in the longitudinal direction thereof when in the free state and is capable of elastically undergoing compression deformation in the diametrical direction thereof, the conduit is received in the groove of the case that has the portion with the constant groove width in at least part of the groove in the longitudinal direction thereof, while remaining compression-deformed in the abovementioned diametrical direction. Specifically, the conduit is arranged between the light diffusion block for light projection that has the light projection surface positioned on the same plane as one of the two side walls of the groove on the one axis line which is orthogonal to the two side walls of the groove at the portion with the constant groove width; and the light diffusion block for light reception that has the light reception surface which is positioned on the same plane as the other of the two side walls on the axis line and faces the light projection surface with a given distance equal to the groove width being provided between the light reception surface and the light projection surface. Next, the transmission lights with the two types of wavelengths that have been supplied from the light emitting element to the given region which is part of the light projection surface of the light diffusion block for light projection and is centered about the axis line, are projected onto the conduit from such light projection surface on the axis line before passing through the conduit wall of the conduit and the water or fluid running thereinside, the two types of wavelengths being the first wavelength that yields an extremely small or absolutely zero absorbance to water but a high absorbance to the fluid, and the second wavelength that yields a high absorbance to water but an extremely low absorbance to the fluid. These transmission lights are then supplied to the light receiving element after being received from the given region which is part of the light reception surface of the light diffusion block for light reception and is centered about the axis line, the light reception surface facing the light projection surface on the axis line with the given distance being provided therebetween.

Moreover, either after or before the aforementioned step, instead of the conduit, a reference block for calibration is to be disposed between the light projection surface of the light diffusion block for light projection and the light reception surface of the light diffusion block for light reception on the axis line. Next, the transmission lights with the two types of wavelengths that have been supplied, in a similar manner as the case involving a conduit, from the light emitting element to the given region which is part of the light diffusion block for light projection and is centered about the axis line are projected from the light projection surface onto the reference block on the axis line before passing through such reference block. These transmission lights are then supplied to the light receiving element after being received from the given region which is part of the light reception surface of the light diffusion block for light reception and is centered about the axis line.

Further, via the signal from the light receiving element, the transmission light intensity measuring unit measures the intensities of the transmission lights with the two types of wavelengths that have been supplied from the light emitting element, projected from the light projection surface along the axis line, received from the light reception surface on the axis line, and then supplied to the light receiving element. Next, the fluid concentration output unit obtains and outputs the concentration of the fluid flowing inside the conduit based on the intensities of the transmission lights with the two types of wavelengths that have passed through the conduit between the light projection surface and the light reception surface, and on separately obtained input intensities of the transmission lights of the two types of wavelengths to the conduit, in accordance with, for example, the Lambert-Beer law, the separately obtained input intensities being obtained from the intensities of the transmission lights with the two types of wavelengths that have passed through the reference block between the light projection surface and the light reception surface.

Thus, according to the fluid concentration measuring device of this invention, a light transmissive conduit such as a resin tube made of vinyl chloride or the like that has a uniform wall thickness and outer diameter in the longitudinal direction when in a free state and is capable of deforming in the diametrical direction, is received in a groove of a case in such a manner that the conduit is compression-deformed in the diametrical direction with a passage being left thereinside; a light projection surface of a light diffusion block for light projection, a light reception surface of a light diffusion block for light reception, and a reference block are alternately arranged on one axis line in such a manner that the light projection surface and the light reception surface face each other with a given distance being provided therebetween, and the reference block is disposed between these light projection surface and the light reception surface; and transmission lights with two types of wavelengths yielding significantly different absorbances are applied to water and a fluid that flow inside the conduit. Thus, the concentration of a fluid inside the conduit can be easily and highly precisely measured even when there are scars on the surface of the conduit, and the removal of the conduit from the groove can also be performed easily.

Here, in the fluid concentration measuring device of this invention, the abovementioned fluid may be blood flowing inside the conduit. It is preferred if the device is configured in this manner, because the concentration of the blood that is collected from an examinee's blood vessel with a blood collection needle for the purpose of, for example, dialysis and flows in the conduit can be easily and continuously measured.

In such case, in the fluid concentration measuring device of this invention, the two types of wavelengths may be set in the vicinity of 805 nm which is a wavelength that is either absorbed in an extremely small amount or not absorbed at all into water, but is capable of being absorbed into blood in such a manner that absorption levels are similar in both oxygenated hemoglobin and deoxygenated (reduced) hemoglobin; and in the vicinity of 1,450 nm which is a wavelength that yields a high absorbance to water. It is preferred if the device is configured in this manner, because the concentration of the blood flowing inside the conduit can be easily and highly precisely measured in a way where there are no changes caused by the oxygenation and deoxygenation of hemoglobin as compared to when employing other wavelengths.

Further, in the fluid concentration measuring device of this invention, it may be that at least one of the light emitting element and the light receiving element has two elements that are aligned and positioned on the one axis line and emit or receive the lights with the two types of wavelengths on the axis line. It is preferred if the device is configured in this manner, because the light axes of the lights with the two types of wavelengths can be accurately matched.

Furthermore, in the fluid concentration measuring device of this invention, it may be that at least one of the light emitting element and the light receiving element has one element that is positioned on the one axis line and emits or receives a light of one of the two types of wavelengths on the axis line; and one element that emits or receives a light of the other of the two types of wavelengths via reflection by a dichroic mirror positioned on the one axis line. It is preferred even when the device is configured in this manner, because the axis lines of the lights with the two types of wavelengths can be accurately matched.

Furthermore, in the fluid concentration measuring device of this invention, there may be provided a light transmissive reference block that is detachably disposed between the light projection surface and the light reception surface and is in close contact with the light projection surface and the light reception surface. In this way, there can be obtained the intensities of the transmission lights with the two types of wavelengths that are projected from the light projection surface.

In such case, the reference block may be received in the case in such a manner that the reference block is capable of moving between a use position that is inside the groove and crosses the axis line and a standby position that is outside the groove. It is preferred if the device is configured in this manner, because not only the handling of the fluid concentration measuring device can be made easy, but the reference block can be prevented from being lost when not used.

### Brief Description of Drawings

[FIGs. 1] (a) and (b) are respectively an exterior perspective view and a partially cut-out perspective view that show the configuration of a blood concentration measuring device as one embodiment of the fluid concentration measuring device of this invention, where shown in both FIGs.1(a) and 1(b) is a state in which a resin tube as a conduit is disposed in a groove of a case, and a reference block is retracted in a standby position in the case.
[FIGs.2] (a) and (b) are another set of exterior perspective view and partially cut-out perspective view that show the configuration of the blood concentration measuring device of the above embodiment, where shown in both
FIGs.2(a) and 2(b) is a state in which the reference block has been brought to a use position in the groove of the case.
[FIGs.3] (a), (b), and (c) are cross-sectional views respectively and schematically showing three types of configurations using a light-emitting diode as a light emitting element in the blood concentration measuring device of the above embodiment.
[FIGs.4] (a) and (b) are cross-sectional views respectively and schematically showing two types of configurations using a laser diode as the light emitting element in the blood concentration measuring device of the above embodiment.
[FIGs.5] (a) and (b) are such that FIG.5(a) is a cross-sectional view schematically showing a modified example of the configuration shown in FIG.3(b), in which a bandpass filter is used; and FIG.5(b) is an explanatory diagram showing a correlation between the wavelength and intensity of a transmission light of such bandpass filter.
[FIGs.6] (a) and (b) are cross-sectional views respectively and schematically showing two types of configurations using a light sensor as a light receiving element in the blood concentration measuring device of the above embodiment.
[FIGs.7] (a) and (b) are cross-sectional views respectively and schematically showing another two types of configurations using a light sensor as the light receiving element in the blood concentration measuring device of the above embodiment.
[FIGs.8] (a) to (e) are explanatory diagrams showing the procedures for measuring blood concentration using the blood concentration measuring device of the above embodiment.
[FIG.9] is a characteristic chart showing a correlation between the wavelength of a transmission light and absorbance with regard to blood and water.

### Description of Embodiments

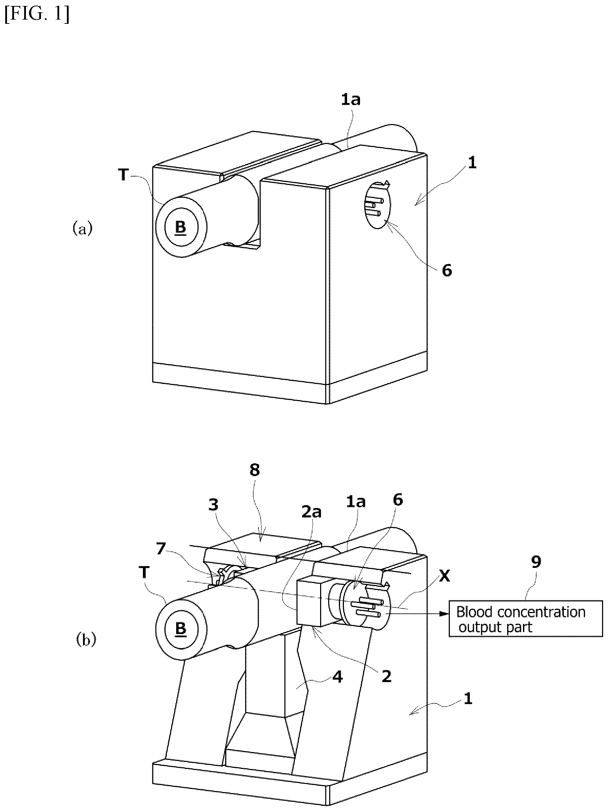

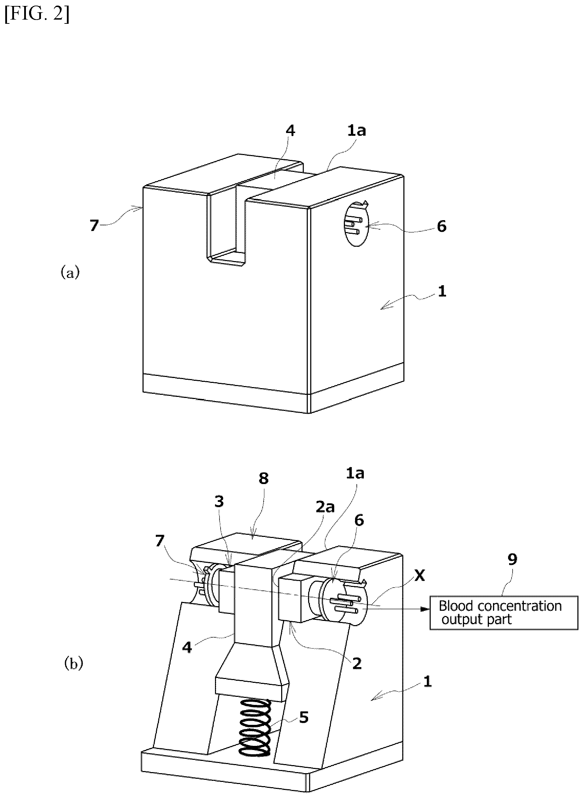

An embodiment of the present invention is described in detail hereunder with reference to drawings. FIGs.1(a) and 1(b) are respectively an exterior perspective view and a partially cut-out perspective view that show the configuration of a blood concentration measuring device as one embodiment of the fluid concentration measuring device of this invention, where shown in both FIGs.1(a) and 1(b) is a state in which a resin tube as a conduit is disposed in a groove of a case, and a reference block is retracted in a standby position in the case. Further, FIGs.2(a) and 2(b) are another set of exterior perspective view and partially cut-out perspective view that show the configuration of the blood concentration measuring device of the above embodiment, where shown in both FIGs.2(a) and 2(b) is a state in which the reference block has been brought to a use position in the groove of the case.

As shown in these drawings, the blood concentration measuring device of this embodiment is a device for measuring the concentration of blood B flowing inside a resin tube T that is made of, for example, vinyl chloride, the resin tube T serving as a light transmissive conduit that has a uniform wall thickness and outer diameter in the longitudinal direction thereof when in a free state and is capable of elastically undergoing compression deformation in the diametrical direction thereof. This blood concentration measuring device includes a case 1 having a groove 1a that is extended in a given direction and receives the resin tube T in such a manner that the resin tube T is compression-deformed in the diametrical direction with a passage being left thereinside, the groove 1a having, for example, a toppled U-shaped cross section and a constant width L in at least part of the groove in the longitudinal direction thereof, where in this embodiment, the entire groove substantially has a constant width L; a light diffusion block for light projection 2 that is formed into the shape of, for example, a rectangular thick plate and has a light projection surface 2a positioned on the same plane as one of two side walls of the groove 1a on one axis line X which is orthogonal to the two side walls of the groove 1a at a longitudinal central portion of the groove 1a; and a light diffusion block for light reception 3 that has, for example, the same shape and dimensions as the light diffusion block for light projection 2 and has a light reception surface 3a which is positioned on the same plane as the other of the two side walls on the axis line X and faces the light projection surface 2a with a given distance equal to the groove width L being provided between the light reception surface 3a and the light projection surface 2a.

Here, an inorganic diffusion plate using a microlens array made of glass or quartz is suitable for use as the light diffusion block for light projection 2 and the light diffusion block for light reception 3 when a later-described transmission light is a light from a laser diode, whereas an organic diffusion plate using a microlens array made of acryl or polyurethane is suitable for use as the light diffusion block for light projection 2 and the light diffusion block for light reception 3 when the transmission light is a light from a light-emitting diode.

Further, the blood concentration measuring device of this embodiment has a reference block 4 that is made of a light transmissive material such as vinyl chloride and is received in the case 1 in such a manner that the reference block 4 is capable of ascending and descending therein and is detachably disposed between the light projection surface 2a of the light diffusion block for light projection 2 and the light reception surface 3a of the light diffusion block for light reception 3, in which the reference block 4 is in close contact with these light projection surface 2a and light reception surface 3a when in the use position where the reference block 4 has ascended to between the two side walls of the groove 1a, and the reference block 4 is positioned detached from between the two side walls of the groove 1a when in the standby position where the reference block 4 has descended from the use position; and an elastic member 5 that always slightly biases the reference block 4 toward the use position and is compressed and received in a concave portion formed in a flared lower part of the reference block 4 when the resin tube T is compressed and disposed in the groove 1a. Here, by forming the reference block 4 with an easily deformable material, the reference block 4 can be easily brought into close contact with the light projection surface 2a and the light reception surface 3a when expanded in the thickness direction as a result of being compressed in the height direction or longitudinal direction at the use position without having to employ significantly high-precision dimensions.

Moreover, the blood concentration measuring device of this embodiment has, in the case 1, a light emitting element(s) 6 that is configured to supply transmission lights having two types of wavelengths to a given region which is part of the light projection surface 2a of the light diffusion block for light projection 2 and is centered about the axis line X, the two types of wavelengths being a first wavelength λ1 that yields an extremely small or absolutely zero absorbance to water W but a high absorbance to blood B, and a second wavelength λ2 that yields a high absorbance to water W, but an extremely low absorbance to blood B; and a light receiving element 7 that is configured to be supplied with the transmission lights P having the two types of wavelengths λ1 and λ2 from a given region which is part of the light reception surface 3a of the light diffusion block for light reception 3 and is centered about the axis line X. Here, the sizes of the given regions of the light projection surface 2a and the light reception surface 3a do not matter so long as they are aligned in the direction of the axis line X with the compression-deformed part of the resin tube T being sandwiched therebetween, and these given regions may be smaller than the compression-deformed part of the resin tube T; however, if the given regions are of sizes closer to the width of the compression-deformed part of the resin tube T, even when there are scars on the surface of the compression-deformed part, the impact of such scars on transmission light intensity can be more alleviated.

In addition, the blood concentration measuring device of this embodiment has a transmission light intensity measuring part 8 as a transmission light intensity measuring unit that is configured to measure, via an electric signal(s) from the light receiving element 7, intensities P of the transmission lights with the two types of wavelengths λ1 and λ2 that are supplied from the light emitting element 6, projected from the light projection surface 2a of the light diffusion block for light projection 2, received from the light reception surface 3a of the light diffusion block for light reception 3, and then supplied to the light receiving element 7; and a normal microcomputer-equipped blood concentration output part 9 as a fluid concentration output unit that is configured to obtain and output the concentration of blood B in the resin tube T based on the intensities P of the transmission lights with the two types of wavelengths that have passed through the reference block 4 or the tube wall of the resin tube T and the blood B thereinside between the light projection surface 2a and the light reception surface 3a.

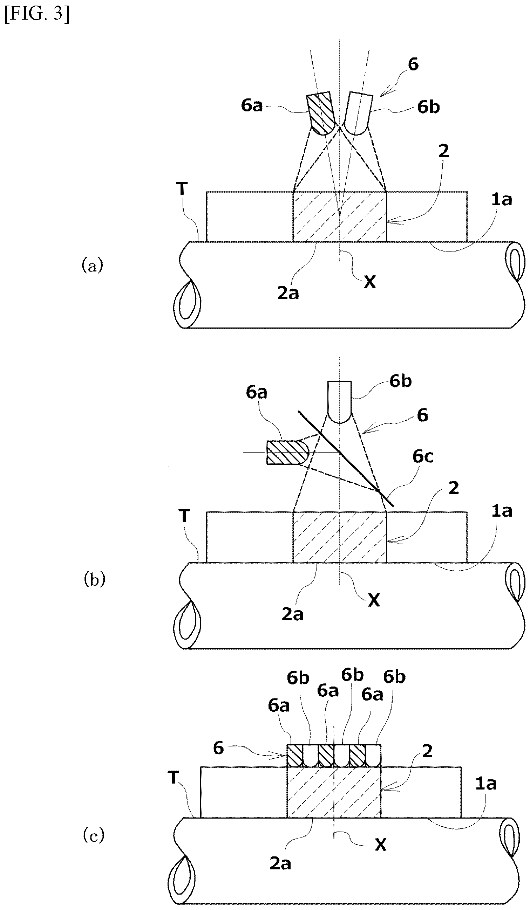

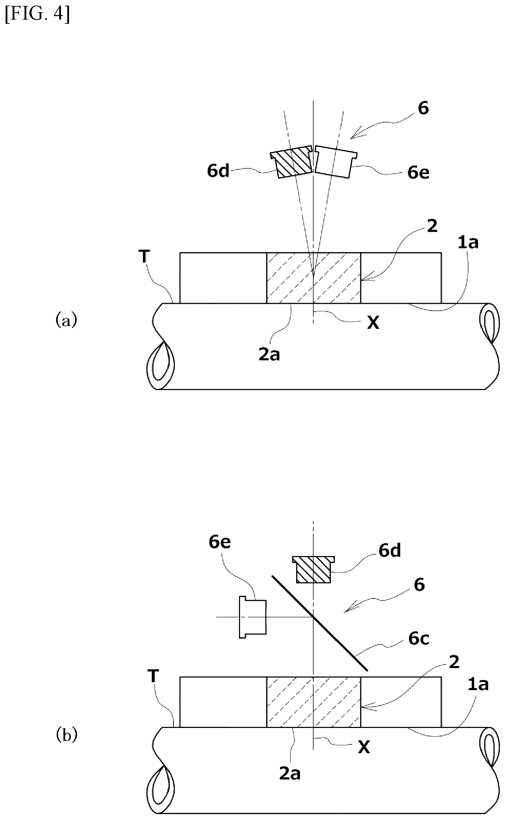

FIGs.3(a), 3(b), and 3(c) are cross-sectional views respectively and schematically showing the configurations of three types of light projection parts using a light-emitting diode as the light emitting element in the blood concentration measuring device of the above embodiment; FIGs. 4(a) and 4(b) are cross-sectional views respectively and schematically showing the configurations of two types of light projection parts using a laser diode as the light emitting element in the blood concentration measuring device of the above embodiment.

In an example shown in FIG.3(a), transmission lights from an LED (light-emitting diode) 6a that emits the light of the wavelength λ1 in a direction oblique to the axis line X, and from an LED (light-emitting diode) 6b that is positioned symmetrical to the LED 6a across the axis line X and emits the light of the wavelength λ2, are supplied to a given region which is part of the resin tube T-abutted light projection surface 2a of the light diffusion block for light projection 2 and is centered about the axis line X, in a manner such that these transmission lights are uniformly diffused through the light diffusion block for light projection 2.

In an example shown in FIG.3(b), a transmission light from the LED (light-emitting diode) 6a that emits the light of the wavelength λ1 on the axis line X, and a transmission light established by having the LED (light-emitting diode) 6b emit the light of the wavelength λ2 at a position deviated from the axis line X and then changing the direction of this light to that matching the axis line X via a dichroic mirror 6c, are supplied to the given region which is part of the resin tube T-abutted light projection surface 2a of the light diffusion block for light projection 2 and is centered about the axis line X, in a manner such that these transmission lights are uniformly diffused through the light diffusion block for light projection 2.

Further, in an example shown in FIG.3(c), transmission lights from the multiple LEDs (light-emitting diodes) 6a and 6b that respectively emit the light of the wavelength λ1 and the light of the wavelength λ2 and are alternately aligned on a surface opposite to the resin tube T-abutted light projection surface 2a of the light diffusion block for light projection 2, are supplied to the given region of the light projection surface 2a that is centered about the axis line X, in a manner such that these transmission lights are uniformly diffused through the light diffusion block for light projection 2.

In an example shown in FIG.4(a), instead of the LEDs 6a and 6b used in the example shown in FIG.3(a), there are arranged an LD (laser diode) 6d that emits a laser light of the wavelength λ1 and an LD (laser diode) 6e that emits a laser light of the wavelength λ2, in a similar manner as the LEDs 6a and 6b, where transmission lights of the laser lights therefrom are uniformly diffused through the light diffusion block for light projection 2 and supplied to the given region of the light projection surface 2a that is centered about the axis line X.

Further, in an example shown in FIG.4(b), instead of the LEDs 6a and 6b used in the example shown in FIG.3(b), there are arranged the LD (laser diode) 6d that emits the laser light of the wavelength λ1 and the LD (laser diode) 6e that emits the laser light of the wavelength λ2, in a similar manner as the LEDs 6a and 6b. Here, a transmission light from the LD 6d that emits the laser light of the wavelength λ1 on the axis line X, and a transmission light established by having the LD 6e emit the laser light of the wavelength λ2 at a position deviated from the axis line X and then changing the direction of this laser light to that matching the axis line X via the dichroic mirror 6c, are uniformly diffused through the light diffusion block for light projection 2 and supplied to the given region of the light projection surface 2a that is centered about the axis line X.

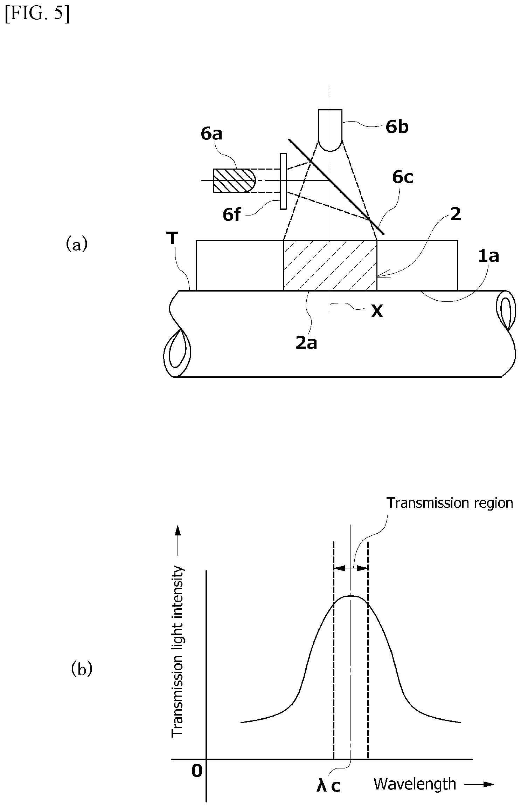

FIG.5(a) is a cross-sectional view schematically showing a modified example of the configuration shown in FIG.3(b), in which a bandpass filter is used; FIG.5(b) is an explanatory diagram showing a correlation between the wavelength and intensity of a transmission light of such bandpass filter. In this modified example, a bandpass filter 6f having a given transmission region with 1,450 nm being a center wavelength λc as shown in FIG.5(b), is disposed in the light projection part 6 in such a manner that the bandpass filter 6f is arranged between the LED (light-emitting diode) 6a and the dichroic mirror 6c that are shown in FIG.3(b), whereby lights with unwanted wavelengths that are emitted from the LED 6a are eliminated.

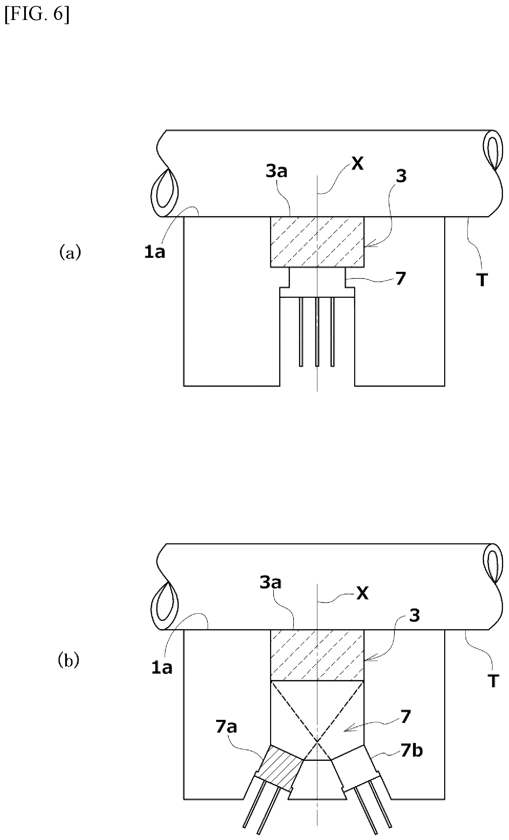

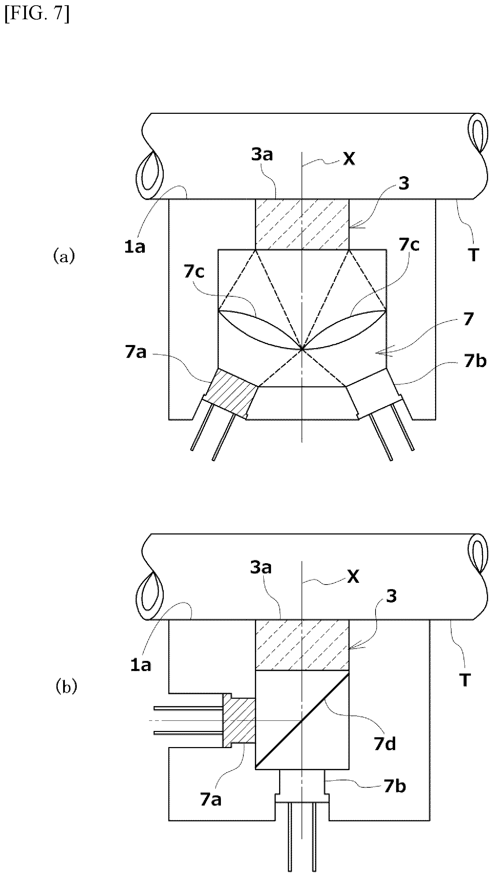

FIGs.6(a) and 6(b) are cross-sectional views respectively and schematically showing the configurations of two types of light reception parts using a light sensor as the light receiving element in the blood concentration measuring device of the above embodiment. Further, FIGs.7(a) and 7(b) are cross-sectional views respectively and schematically showing the configurations of another two types of light reception parts using a light sensor as the light receiving element in the blood concentration measuring device of the above embodiment.

In an example shown in FIG.6(a), light reception takes place in a given region which is part of the resin tube T-abutted light reception surface 3a of the light diffusion block for light reception 3 and is centered about the axis line X, whereby the light of the wavelength λ1 and the light of the wavelength λ2 that have been uniformly diffused through the light diffusion block for light reception 3 are then received by a light sensor 7 having, in the same package and in an aligned manner on the axis line X, a light receiving element receiving the light of the wavelength λ1 and a light receiving element receiving the light of the wavelength λ2, and the light sensor 7 further converts these lights of the wavelengths λ1 and λ2 into electric signals and outputs them. Here, the light sensor 7 may be one having in its package a light receiving element that has sensitivities to both the light of the wavelength λ1 and the light of the wavelength λ2.

Further, in an example shown in FIG.6(b), light reception takes place in the given region which is part of the resin tube T-abutted light reception surface 3a of the light diffusion block for light reception 3 and is centered about the axis line X, whereby the light of the wavelength λ1 and the light of the wavelength λ2 that have been uniformly diffused through the light diffusion block for light reception 3 are respectively converted into electric signals by a light sensor 7a that receives the light of the wavelength λ1 in a direction oblique to the axis line X, and a light sensor 7b that is positioned symmetrical to the light sensor 7a across the axis line X and receives the light of the wavelength λ2, where the electric signals are then respectively outputted therefrom.

In an example shown in FIG.7(a), light reception takes place in the given region which is part of the resin tube T-abutted light reception surface 3a of the light diffusion block for light reception 3 and is centered about the axis line X, whereby the light of the wavelength λ1 and the light of the wavelength λ2 that have been uniformly diffused through the light diffusion block for light reception 3 are respectively enhanced by a condenser lens 7c before being received by the light sensor 7a that receives the light of the wavelength λ1 in a direction oblique to the axis line X, and the light sensor 7b that is positioned symmetrical to the light sensor 7a across the axis line X and receives the light of the wavelength λ2, and the light sensors further convert these lights into electric signals and output them.

Further, in an example shown in FIG.7(b), instead of the LED 6a that emits the light of the wavelength λ1 and the LED 6b that emits the light of the wavelength λ2 as is the case with the configuration shown in FIG.5(a), there are arranged the light sensor 7a that receives the light of the wavelength λ1 and the light sensor 7b that receives the light of the wavelength λ2 without employing the bandpass filter 6f. Here, light reception takes place in the given region which is part of the resin tube T-abutted light reception surface 3a of the light diffusion block for light reception 3 and is centered about the axis line X, whereby the light of the wavelength λ1 and the light of the wavelength λ2 that have been uniformly diffused through the light diffusion block for light reception 3 are respectively received by the light sensor 7a after having the direction of the light changed by a dichroic mirror 7d, and by the light sensor 7b after allowing the light to pass through such dichroic mirror 7d, and the lights received are then respectively converted into electric signals by these light sensors 7a and 7b, where the electric signals are then respectively outputted therefrom.

Using a normal microcomputer, the blood concentration output part 9 shown in FIGs.1(b) and 2(b) obtains and outputs the concentration of blood B in the resin tube T based on the intensities P of the transmission lights with the two types of wavelengths that have passed through the reference block 4 or the tube wall of the resin tube T and the blood B thereinside between the light projection surface 2a and the light reception surface 3a.

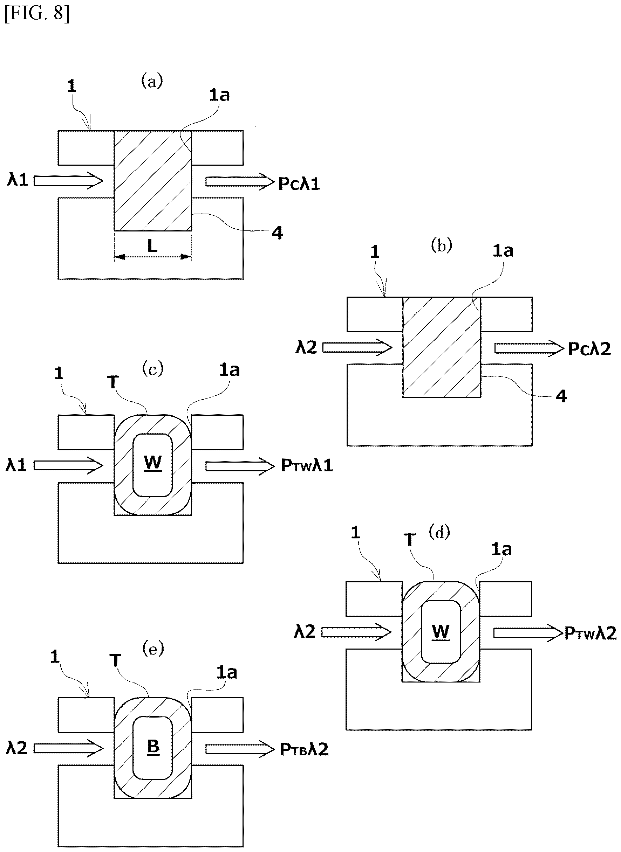

FIGs.8(a) to 8(e) are explanatory diagrams showing the procedures for measuring blood concentration using the blood concentration measuring device of the above embodiment. At first, as shown in FIG.8(a), the reference block 4 is placed in the use position inside the groove 1a of the case 1, followed by supplying the light of the wavelength λ1 from the light emitting element 6 so as to project the light to the reference block 4 from the light projection surface 2a of the light diffusion block for light projection 2, and then allow the light to pass through the reference block 4 before being received from the light reception surface 3a of the light diffusion block for light reception 3, whereby an intensity Pcλ1 of the transmission light of the wavelength λ1 that has been supplied to the light receiving element 7 is measured via the electric signal from the light receiving element 7.

Next, as shown in FIG.8(b), with the reference block 4 being placed in the use position inside the groove 1a of the case 1, the light of the wavelength λ2 is supplied from the light emitting element 6 so as to project the light to the reference block 4 from the light projection surface 2a of the light diffusion block for light projection 2, and then allow the light to pass through the reference block 4 before being received from the light reception surface 3a of the light diffusion block for light reception 3, whereby an intensity Pcλ2 of the transmission light of the wavelength λ2 that has been supplied to the light receiving element 7 is measured via the electric signal from the light receiving element 7.

Next, as shown in FIG.8(c), the reference block 4 is to be positioned outside the groove 1a by being, for example, pushed into the standby position in the lower area of the groove 1a against the elastic force of the elastic member 5. Instead, the resin tube T is pushed into the groove 1a of the case 1 so that the resin tube T is sandwiched by the two side walls of the groove 1a as well as the light projection surface 2a of the light diffusion block for light projection 2 and the light reception surface 3a of the light diffusion block for light reception 3 that are positioned on the same planes as these side walls, thus causing the resin tube T to be compression-deformed in the diametrical direction with a passage being left thereinside. Water W is then passed through the inner portion of the resin tube T of such state, and the light of the wavelength λ1 is supplied from the light emitting element 6 so as to project the light to the resin tube T from the light projection surface 2a of the light diffusion block for light projection 2, and then allow the light to pass through the tube wall of the resin tube T and the water W thereinside before being received from the light reception surface 3a of the light diffusion block for light reception 3, whereby an intensity PTWλ1 of the transmission light of the wavelength λ1 that has been supplied to the light receiving element 7 is measured via the electric signal from the light receiving element 7.

Next, as shown in FIG.8(d), with water W still being passed through the passage inside the resin tube T that is positioned in the groove 1a of the case 1 and is compression-deformed in the diametrical direction, the light of the wavelength λ2 is supplied from the light emitting element 6 so as to project the light to the resin tube T from the light projection surface 2a of the light diffusion block for light projection 2, and then allow the light to pass through the tube wall of the resin tube T and the water W thereinside before being received from the light reception surface 3a of the light diffusion block for light reception 3, whereby an intensity PTWλ2 of the transmission light of the wavelength λ2 that has been supplied to the light receiving element 7 is measured via the electric signal from the light receiving element 7.

Further, as shown in FIG.8(e), with the resin tube T being positioned in the groove 1a of the case 1 and compression-deformed in the diametrical direction, water W is changed to blood B, and blood B as a concentration measurement target fluid is passed through the passage thereinside instead. The light of the wavelength λ2 is supplied from the light emitting element 6 so as to project the light to the resin tube T from the light projection surface 2a of the light diffusion block for light projection 2, and then allow the light to pass through the tube wall of the resin tube T and the blood B thereinside before being received from the light reception surface 3a of the light diffusion block for light reception 3, whereby an intensity PTBλ2 of the transmission light of the wavelength λ2 that has been supplied to the light receiving element 7 is measured via the electric signal from the light receiving element 7. Here, the emission intensity of each wavelength at the time of measuring these transmission light intensities remains unchanged.

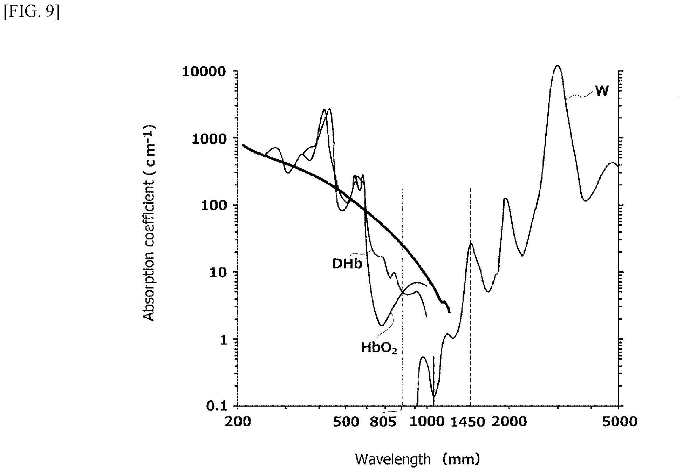

FIG.9 is a characteristic chart showing a correlation between the wavelength of a transmission light and an absorption coefficient with regard to water W as well as oxygenated hemoglobin HbO₂ and deoxygenated hemoglobin DHb in blood B. As is clear from this chart, the light of the wavelength λ1 is one with a center wavelength of 1,450 nm, which is capable of being absorbed into both water W and blood B; the light of the wavelength λ2 is one with a center wavelength of 805 nm, which is either absorbed in an extremely small amount or not absorbed at all into water W, but is capable of being absorbed into blood B in such a manner that the absorption levels are similar in both oxygenated hemoglobin HbO₂ and deoxygenated (reduced) hemoglobin DHb. Here, there are no particular limitations on the ingredients of water W as long as it is substantially transparent to the extent that it exhibits the kind of correlation between the wavelength of a transmission light and an absorption coefficient as shown in the chart; water W may, for example, be saline.

Therefore, based on these transmission light intensity data, the blood concentration output part 9 is able to obtain the concentration of blood B via the following procedures.
(1) Since the absorption rate (absorption coefficient) and reflection loss of the reference block 4 with regard to the wavelengths λ1 and λ2 are fixed values, input intensities Iλ1 and Iλ2 of the wavelengths λ1 and λ2 from the light projection surface 2a to the reference block 4 can be obtained.
(2) The transmission light intensity PTWλ1 of the resin tube T through which water W is passed under the wavelength λ1 includes attenuation and reflection loss attributed to the tube wall and the water W inside the tube. In contrast, the transmission light intensity PTWλ2 of the resin tube T through which water W is passed under the wavelength λ2 substantially and only includes the attenuation and reflection loss that is attributed to the tube wall. Thus, by taking an attenuation ratio between the wavelength λ1 and the wavelength λ2 into consideration, only an attenuation quantity attributed to the water W inside the resin tube T is obtained, and the optical path length of the fluid, i.e., blood B inside the resin tube T is obtained from the absorption rate (absorption coefficient) of water W.
(3) The intensity PTBλ2 of the transmission light with the wavelength λ2 that is supplied to the light receiving element 7 includes attenuation attributed to the tube wall of the resin tube T and attenuation attributed to the blood B thereinside. Since the attenuation quantity that is substantially only attributed to the tube wall has already been obtained in the above (2), by subtracting such attenuation quantity only attributed to the tube wall from the intensity PTBλ2 of the transmission light with the wavelength λ2, an attenuation quantity only attributed to the blood B in the resin tube T can be obtained. And, since the optical path length of the blood B in the resin tube T has also already been obtained in the above (2), the concentration of the blood B in the resin tube T can be obtained from these values by performing calculations based on the Lambert-Beer law (e.g., see the publication of Japanese Patent No.6246793 previously disclosed under the names of the applicants of the present application).

The present invention has thus far been described based on the embodiment shown in the drawings; the fluid concentration measuring device of this invention shall not be limited to the above embodiment, but may be appropriately subjected to modifications within the scope described in the claims. For example, the cross-sectional shape of the groove 1a may be a U shape or the like instead of a toppled U shape. Further, the reference block 4 may be configured in such a way that instead of moving the reference block 4 between the use position and the standby position in the case 1, the reference block 4 may be placed in the use position in the groove 1a at the time of use and then removed from the case 1 when not in use.

Further, the blood concentration output part 9 may be configured to obtain the concentration of blood B from the transmission light intensity data, using a correspondence table that has been previously prepared via calculations based on the Lambert-Beer law. Moreover, the concentration measurement target fluid of the fluid concentration measuring device of this invention may be a drug solution or the like instead of blood B.

### Industrial Applicability

In this way, according to the fluid concentration measuring device of this invention, a light transmissive conduit such as a resin tube made of vinyl chloride or the like that has a uniform wall thickness and outer diameter in the longitudinal direction when in a free state and is capable of deforming in the diametrical direction, is received in a groove of a case in such a manner that the conduit is compression-deformed in the diametrical direction with a passage being left thereinside; a light projection surface of a light diffusion block for light projection, a light reception surface of a light diffusion block for light reception, and a reference block are alternately arranged on one axis line in such a manner that the light projection surface and the light reception surface face each other with a given distance being provided therebetween, and the reference block is disposed between these light projection surface and light reception surface; and transmission lights with two types of wavelengths yielding significantly different absorbances are applied to water and a fluid that flow inside the conduit. Thus, the concentration of a fluid inside the conduit can be easily and highly precisely measured even when there are scars on the surface of the conduit, and the removal of the conduit from the groove can also be performed easily.

### Reference Signs List

1 Case
1a Groove
2 Light diffusion block for light projection
2a Light projection surface
3 Light diffusion block for light reception
3a Light reception surface
4 Reference block
5 Elastic member
6 Light emitting element
6a, 6b LED
6c Dichroic mirror
6d, 6e LD
6f Bandpass filter
7 Light receiving element
7a, 7b Light sensor
7c Condenser lens
7d Dichroic mirror
8 Transmission light intensity measuring part
9 Blood concentration output part
B Blood
T Resin tube
W Water
X Axis line

## Claims

1. A fluid concentration measuring device for measuring the concentration of a fluid flowing in a light transmissive conduit that has a uniform wall thickness and outer diameter in a longitudinal direction thereof when in a free state and is elastically compression-deformable in a diametrical direction thereof, comprising:
a case having a groove that is extended in a given direction and has a portion with a constant groove width in at least a part of the groove in a longitudinal direction thereof, the portion with a constant groove width receiving the conduit in such a manner that the conduit is compression-deformed in the diametrical direction with a passage being left thereinside;
a light diffusion block for light projection that has a light projection surface positioned on the same plane as one of two side walls of the groove on one axis line which is orthogonal to the two side walls of the groove at the portion with the constant groove width;
a light diffusion block for light reception that has a light reception surface which is positioned on the same plane as the other of the two side walls on the axis line and faces the light projection surface with a given distance equal to the groove width being provided between the light reception surface and the light projection surface;
a light emitting element that is configured to supply transmission lights having two types of wavelengths to a given region which is a part of the light projection surface of the light diffusion block for light projection and is centered about the axis line, the two types of wavelengths being a first wavelength that yields an extremely small or absolutely zero absorbance to water but a high absorbance to the fluid, and a second wavelength that yields a high absorbance to water;
a light receiving element that is configured to be supplied with the transmission lights having the two types of wavelengths from a given region which is a part of the light reception surface of the light diffusion block for light reception and is centered about the axis line;
a transmission light intensity measuring unit that is configured to measure, via a signal from the light receiving element, the intensities of the transmission lights with the two types of wavelengths that are supplied from the light emitting element, projected from the light projection surface, received from the light reception surface, and then supplied to the light receiving element; and
a fluid concentration output unit that is configured to obtain and output the concentration of the fluid based on the intensities of the transmission lights with the two types of wavelengths that have passed through the conduit between the light projection surface and the light reception surface, and on separately obtained input intensities of the transmission lights of the two types of wavelengths to the conduit.

2. The fluid concentration measuring device according to claim 1, wherein the fluid is blood flowing inside the conduit.

3. The fluid concentration measuring device according to claim 2, wherein the two types of wavelengths are set in the vicinity of 805 nm which is a wavelength that is either absorbed in an extremely small amount or not absorbed at all into water, but is capable of being absorbed into blood in such a manner that absorption levels are similar in both oxygenated hemoglobin and deoxygenated hemoglobin; and in the vicinity of 1,450 nm which is a wavelength that yields a high absorbance to water.

4. The fluid concentration measuring device according to any one of claims 1 to 3, wherein at least one of the light emitting element and the light receiving element has two elements that are aligned and positioned on the one axis line and emit or receive the lights with the two types of wavelengths on the axis line.

5. The fluid concentration measuring device according to any one of claims 1 to 3, wherein at least one of the light emitting element and the light receiving element has one element that is positioned on the one axis line and emits or receives a light of one of the two types of wavelengths on the axis line; and one element that emits or receives a light of the other of the two types of wavelengths via reflection by a dichroic mirror positioned on the one axis line.

6. The fluid concentration measuring device according to any one of claims 1 to 3 further comprising a light transmissive reference block that is detachably disposed between the light projection surface and the light reception surface and is in close contact with the light projection surface and the light reception surface.

7. The fluid concentration measuring device according to claim 6, wherein the reference block is received in the case in such a manner that the reference block is capable of moving between a use position that is inside the groove and crosses the axis line and a standby position that is outside the groove.
